# NEW EUROPEAN PATENT SPECIFICATION

(11) **EP 1 766 245 B2**
(45) Date of publication and mention of the opposition decision: **23.03.2022**
(45) Mention of the grant of the patent: 21.02.2018
(21) Application number: 05747227.6
(22) Date of filing: 03.06.2005
(51) Int. Cl.: F04D 29/70, A01K 63/04, C02F 1/32

(54) **A PUMP UNIT**
PUMPENEINHEIT
UNITE DE POMPAGE

(30) Priority: 10.06.2004 GB 0412983; 23.12.2004 US 639711 P
(43) Date of publication of application: 28.03.2007
(60) Divisional application: 17211191.6
(73) Proprietor: EXEL Industries S.A., 51200 Epernay (FR)
(72) Inventor: PATEL, Kaushik, Ishwarbhai, Northwood, Middlesex HA6 1QJ (GB); YUNG, Phong Yui, Poplar, London E14 OSP (GB)
(74) Representative: Faulkner, Thomas John
(86) International application number: PCT/GB2005/002186
(87) International publication number: WO 2005/090782

(56) References cited:
- EP-A- 0 802 163
- EP-A1- 1 068 888
- EP-A1- 1 231 384
- DE-A1- 2 803 064
- GB-A- 2 249 136
- GB-A- 2 307 421
- JP-A- 2000 296 391
- US-A1- 2002 139 865
- US-A1- 2003 062 295

## Description

This invention relates to a pump unit for placing in a pond.

The water in ponds, such as ornamental ponds found in domestic gardens, can become, or give the appearance of being, dirty or polluted. For example, pond water may take on a green colour due to the growth of algae or similar organisms in that water. Pond water may also become dirtied with non-living solid matter.

To overcome this problem, pond filters are available for positioning near to a pond and for receiving a flow of water from the pond. The filters filter water received from the pond and then return the filtered water to the pond. Water is received from the pond along a hose running from a pump and is returned to the pond by way of another hose.

Ultra-violet (UV) filters are also available. These include a UV lamp for exposing water flowing through the UV filter to UV light. The UV filters are also for positioning near to a pond and may be positioned to receive water from a pump and to supply water to a pond filter of the kind just described.

It is an object of this invention to improve the prior art.

DE 2803064 describes a pump used for swimming pool or aquarium cleaning. It has a collection chamber in which a UV lamp may be located.

US2002/0139865 describes a water display system with a germicidal light source. Water in a reservoir may be exposed to radiation from a UV lamp.

JP2000 296391 describes a pond filter comprising a pump, UV lamp, mechanical filtering means and a biological filtering means.

According to a first aspect of this invention, there is provided a pond pump unit for placing in a pond, according to claim 1.

By providing a pump unit that includes both an electrical pump and a UV lamp, a neat and simple unit results that removes the need for a separate, unsightly UV filter situated near to the pond, together with the associated water hoses running to and from the pond. Instead, the UV lamp is placed out of sight in the pond.

The unit may be arranged such that the pump draws water from the pond past the UV lamp and then into an inlet of the pump unit. The unit may be arranged such that the pump draws water from the pond and pumps that water, from an outlet of the pump, past the UV lamp.

The unit may be arranged such that electrical components of the pump are contained in sealed container which is sealed against the ingress of water. The unit may be arranged such that electrical components of the lamp are contained in sealed container which is sealed against the ingress of water.

The unit may be arranged such that electrical components of the pump and of the lamp are contained together in a sealed container in the housing, the sealing being against the ingress of water. All of the electrical components of the pump and the lamp may be contained in the container. This allows the pump and the lamp to be powered by power from a single power lead to the container. Typically, the container is not of one-piece construction and may, instead, be formed from separable components. The sealed container may have a single power lead extending therefrom to supply electrical power to both of the pump and the lamp. The use of a single power lead, rather that a respective lead for each of the pump and the lamp simplifies construction of the unit and also simplifies installation of the unit in a pond. It may also be considered a safer configuration as, the fewer the number of power leads, the less is the likelihood of one being inadvertently damaged, for example by a lawn mower or other garden cutting apparatus, and conductors of the lead being thereby exposed.

A single sealed container is also advantageous in that it can require fewer seals than would, for example, two sealed containers.

The sealed container may be at least partly constituted by a removable cover for the UV lamp, the cover being removable from the remainder of the sealed container. The cover is preferably at least partly of a material, such as quartz, that tends to allow UV light to pass readily therethrough. Preferably the cover is a quartz tube, closed at one end and with mounting structure at the other, open, end for sealedly and releasably mounting the cover to the remainder of the container.

The unit may be arranged such that the major dimension of the UV lamp extends in the direction of water flow therepast. Preferably, where the UV lamp is tubular, the unit is arranged such that water flow past the lamp is substantially axial and, preferably, is all around the lamp in order to maximise exposure of the water to UV light emitted by the lamp.

The unit may be arranged such that the filtering means filters water drawn from the pond before or after that water reaches the pump and/or before or after that water flows past the lamp. The apertures formed in the housing may be slots. The filtering means may include a filter member that is removably locatable in the housing. The filter member may include a mesh member. The mesh member may include a network of strands running in three dimensions between which water is drawn for filtering. The filter member may include a foam member for filtering water drawn therethrough. Preferably, the filtering means is arranged such that water is drawn from the pond therethrough before passing through the pump. This minimises the risk of the pump being clogged by solid matter or of the pump chopping up that matter into pieces. Chopping the matter into pieces is disadvantageous as the pieces may be small enough to avoid being caught by the mechanical filtering means.

The bypass may include a valve. Preferably, the valve is arranged such that at least partial clogging of the filter means increases a pressure difference across the valve such that the valve opens and allows water drawn from the pond to pass therethrough and to thereby bypass at least part of the filter means. Where the filtering means includes a filter member, that member may include a bypass flow path therethrough in which the valve is situated. A bypass member may be provided that defines a bypass flow path with the valve situated therein. The valve may include a thin flap of material that is resiliently biased in a closed position and may be situated at an outlet side of the bypass flow path. The bypass member may be arranged to receive the filter member such that the bypass flow path extends through the member.

The housing may include a lid portion that is removably attachable to the remainder of the housing. The lid portion preferably is adjacent the filter medium such that removal of the lid portion facilitates access to the filter medium for inspection and/or removal thereof. The lid portion may include at least one catch for releasably attaching that portion to the remainder of the housing. The catch may be a sprung catch that is depressible by a user to allow removal of the lid portion.

The unit includes a biological filtering structure arranged with a high surface area to volume ratio and arranged in the housing such that water drawn from the pond is caused to flow over and around the structure. Organisms present in the water will tend to grow on the surface of the structure and to clean water passing thereover and therearound. Preferably, the biological filtering structure is arranged in the housing such that water is drawn thereover and therearound after having passed through the filter means and before passing through the pump.

Preferably, the unit is arranged such that the fountain outlet receives water that has already flowed past the UV lamp. This is advantageous in that water which has been exposed to UV light from the lamp is then dispersed by the fountain outlet about the pond, rather than just in the vicinity of the unit. This tends to reduce the time taken for or, at least increase the likelihood of, substantially all of the water in the pond being drawn through the unit and being exposed to UV light from the lamp.

The variable flow control means may be arranged to be operable between a state in which flow through the bypass outlet is prevented and a state in which all water drawn from the pond is allowed to pass through the bypass outlet, and operable at states therebetween. Preferably, however, the variable flow control means is arranged so as to always allow at least some flow therethrough. This ensures that, should the fountain outlet become clogged, water may still exit the unit. The variable flow control means may be arranged to have the effect of controlling the height of the fountain from the fountain outlet. The variable flow control means may comprise a rotatable ring.

The bypass outlet may be positioned in the unit away from an inlet thereto through which water is drawn from the pond by the electrical pump. The bypass outlet may be positioned away from the mechanical filtering means, and preferably is positioned away from the apertures formed in the housing and through which water is drawn into the unit.

The housing may include outlet structure that at least partly defines the fountain outlet, the outlet structure being moveable relative to the remainder of the housing so as to allow water exiting the unit from the fountain outlet to be directed in a preferred direction. The outlet structure may be so moveable by the provision of a ball joint between the outlet structure and the remainder of the housing.

The unit may be arranged with a base portion and a projecting upper portion upstanding therefrom. The filtering means may conveniently be housed in the base portion, together, optionally, with the pump. The UV lamp may conveniently be housed in the upper portion, with the fountain outlet preferably arranged at the distal end of that portion.

The outlet structure may be removable and an obscuring plate may be provided in the housing to obscure viewing of the UV lamp when the outlet structure is removed.

The ball joint may form part of a ball joint assembly comprising the ball joint and the obscuring plate. The ball joint assembly may be captured in the housing. This can help prevent accidental viewing of the UV lamp. The obscuring plate may be dimensioned so that it is unable to pass through an aperture in the housing through which the ball joint projects. The obscuring plate may be mounted to the ball joint after the ball joint has been mounted in the housing. The obscuring plate may be fixed by a screw to the ball joint. Alternatively the obscuring plate may be clipped to or push fitted to the ball joint.

The unit may comprise a takeoff outlet for supplying water via a hose to a remote outlet. The remote outlet might be used to provide a waterfall or other water feature. The takeoff outlet may be distinct from any fountain or bypass outlet provided. The unit may comprise variable flow control means for variable control of water flow through the takeoff outlet. This variable flow control means may comprise a user operable rotatable ring. The rotatable ring may surround a portion of the housing and may be incomplete. This variable flow control means may comprise a collar control. Preferably the unit is arranged so that the takeoff outlet is positioned in the flow path so that water leaving via the takeoff outlet will have passed the UV lamp and preferably any other filter means included in the unit. The unit may comprise a takeoff outlet conduit providing a passage from the downstream end of the UV lamp to the takeoff outlet. The takeoff outlet conduit may comprise an inlet disposed at the downstream end of the UV lamp. The variable flow control means may comprise a closure member for closing, by controllable degrees, the inlet of the takeoff outlet conduit. The takeoff outlet may disposed towards the base of the unit. The takeoff outlet may be disposed closer to a base of the unit than is the distal end of the UV lamp. This can help with stability.
Where there are variable control means for the takeoff outlet and the fountain bypass outlet, the manually operated actuators, for example rotatable rings/control collars may be disposed adjacent one another. The bypass may be arranged so that water exiting via the bypass passes between the two control rings.

Specific pump units in which the invention is embodied are described now by way of example only and with reference to the accompanying drawings, in which:
Figure 1 is a perspective view of a first pump unit;
Figure 2 is a perspective view of the unit of Figure 1 with part of a housing thereof removed to show internal detail;
Figure 3 is a perspective view of mechanical filtering means of the pump unit of Figure 1;
Figure 4 is another perspective view of the pump unit of Figure 1;
Figure 5 is a sectional view of the pump unit of Figure 1;
Figure 6 is a sectional view of a second pump unit which includes a take off outlet; and
Figure 7 is an exploded view of part of the pump unit of Figure 6 which illustrates a flow control mechanism for the take off outlet.

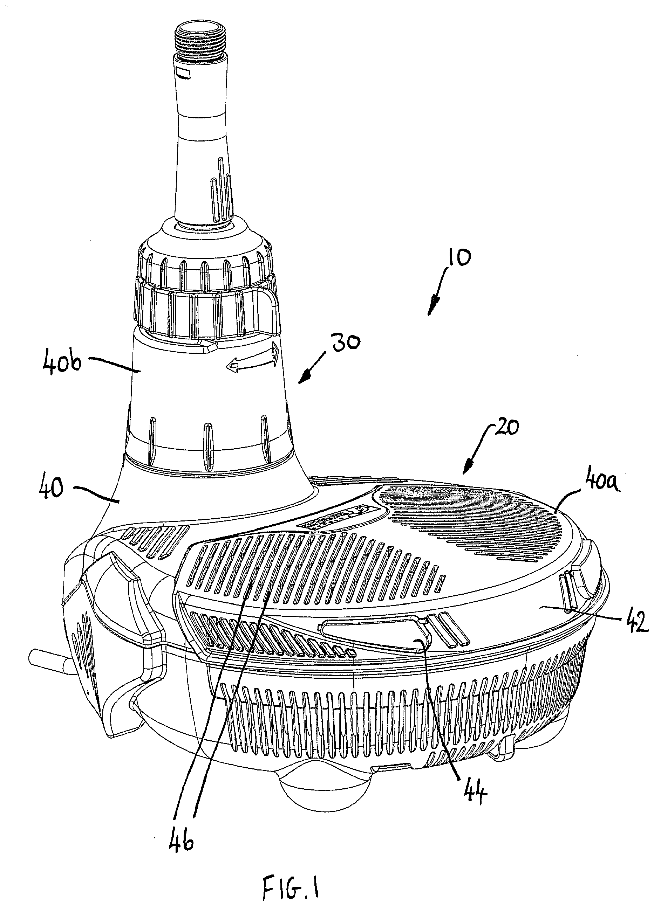

Figure 1 shows a pump unit 10 having a squat cylindrical base portion 20 and a narrower cylindrical upper portion 30 that projects from the base portion 20 so as to be upstanding from it. The upper portion 30 is positioned towards the periphery of the base portion 20. The pump unit 10 is for placing in a pond so as to be submerged in water of the pond.

The pump unit 10 has a plastic housing 40 that houses components (not shown in Figure 1) of the unit 10. The housing 40 is made up of a base housing 40a that is formed to define the outer shape of the base portion 20, and an upper housing 40b that is formed to define the outer shape of the upper portion 30. The majority of the upper part of the base housing 40a forms a removable lid 42 that is removable from the remainder of the base housing 40a. The lid 42 includes two catches (only part of which is visible at 44) that are for releasably securing the lid 42 to the remainder of the base housing 40a. Each of the catches has a respective button portion 44 that projects through an aperture formed in the lid 42 for operation by a user to disengage the respective catch from the remainder of the base housing 40a and thereby allow removal of the lid 42.

The base housing 40a is formed with many apertures through it in the form of slots 46. The slots 46 are formed in the lid 42 and in the side of the base housing 40a adjacent the lid 42.

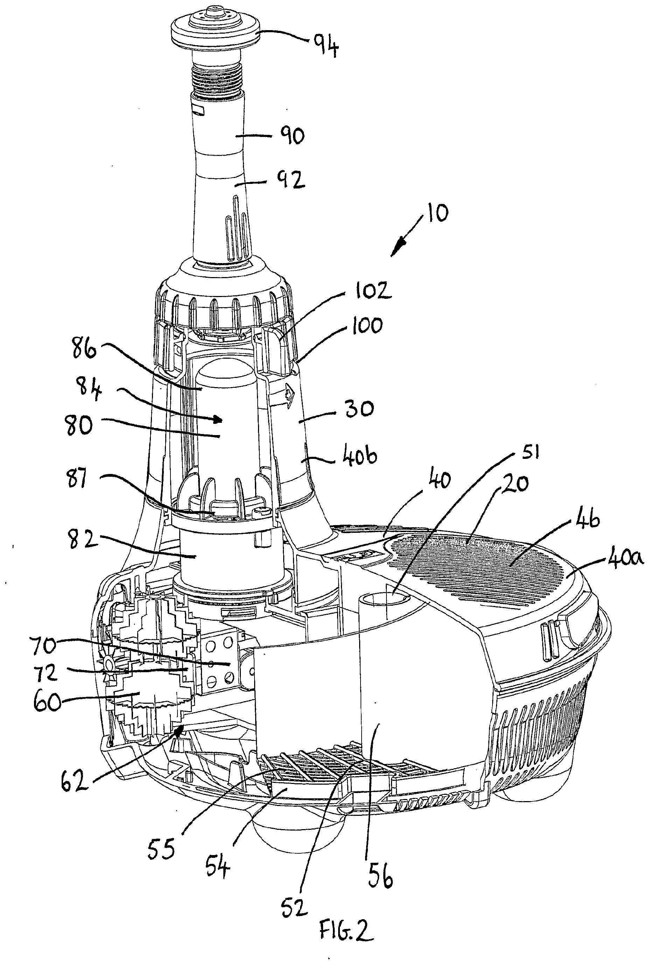

Figure 2 shows the pump unit 10 with part of the housing 40 removed in order to show the inside of the unit 10. With reference to Figure 2, the pump unit 10 includes mechanical filtering means in the form of a foam block 50, biological filtering structure in the form of "biomedia" 60, an electrical pump 70, a UV lamp 80, a fountain outlet 90 and a bypass outlet 100.

The foam block 50 is generally semi-circular in cross-section. The block 50 is arranged in the squat cylindrical base portion 20 against the outside of the base portion 20 and underneath the lid 42. The block 50 is shaped to substantially fill the inside of the unit 10 that is beneath the lid 42. The block 50 is made of a coarse foam-like material that is arranged to mechanically filter water passing through it by straining solid matter from that water.

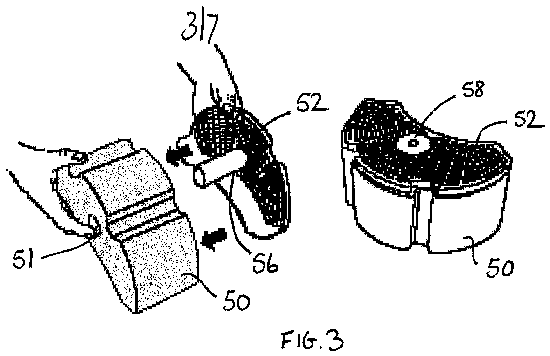

The foam block 50 is supported on a bypass member 52 on which the block 50 is seated. The bypass member 52 is formed of moulded plastic and includes a base portion 54 and a cylindrical tube 56. The base portion 54 is shaped as the base of the block 50 and includes many apertures 55 through it. The base portion 54 also includes a peripheral wall that follows snugly the sides of the block 50. The cylindrical tube 56 is upstanding from the base portion 54 and is accommodated in the block 50 by way of a cylindrical aperture 51 extending through the block 50, from its base to its top. The tube 56 extends through the aperture 51. The foam block 50 and the bypass member 52 are also shown in Figure 3. As shown in Figure 3, the lower end of the tube 56 is provided with a flap valve 58. The valve 58 is arranged to allow liquid flow in a downwards direction only, and only when a pressure differential across the valve (i.e. the difference between pressure at the inlet side of the valve and pressure at the outlet side of the valve) is above a certain pressure difference.

With reference to Figure 2, the unit 10 includes a chamber 62 in the base portion 20 that communicates with the underside of the base portion 54 of the bypass member 52. This chamber 62 is generally underneath and extends to each side of the upper portion 30. The chamber 62 accommodates the biomedia 60. The biomedia 60 are plastic structure with high surface area to volume ratio and, in this embodiment, take the form of six ball-like objects formed of many intersecting, jagged, generally planar surfaces.

The electrical pump 70 is positioned in the housing 40 so as to be in the chamber 62 in which the biomedia 60 are also accommodated. The electrical pump 70, however, is more centrally positioned so as to be underneath the upper portion 30. Thus, the biomedia 60 are positioned to the sides of the electrical pump 70. Casing 72 of the electrical pump 70 forms a barrier at the base of that upper portion 30 between the chamber 62 and the inside of the upper portion 30 of the unit 10. The casing 72 also defines an inlet of the electrical pump (not visible in Figure 2) that extends into the chamber 62 and an outlet (not visible in Figure 2) that extends into the upper portion 30. The electrical pump 70 may be arranged to receive a mains voltage at, for example, 240V AC or 110V AC; or a low voltage at, for example, 24V AC. In this embodiment, the electrical pump 70 operates at a mains voltage.

The UV lamp 80 is positioned inside the upper portion 30 of the pump unit 10 and is an arrangement of several parts. A base 82 of the lamp 80 is joined to the pump casing 72 so as to be an extension of that casing 72 and so as to project therefrom upwards into the upper portion 30. The base 82 houses an electrical socket (not shown in Figure 2) into which a UV bulb 84 of the UV lamp 80 is releasably locatable. The lamp 80 also includes a removable cover in the form of a quartz tube 86, closed at one end and fitted with a bayonet fitting 87 at the other. The bayonet fitting 87 and the base 82 are such that the fitting 87 can be received in the base 82 and twisted into a position in which a watertight seal is created between the base 82 and the fitting 87, and hence also the quartz tube 86. A screw 88 is provided for keeping the bayonet fitting 87, and hence the quartz tube 86, in place. Thus, the quartz tube 86, with the UV bulb 84 inside, project upwardly from the base 82 into and through the upper portion 30 of the unit 10.

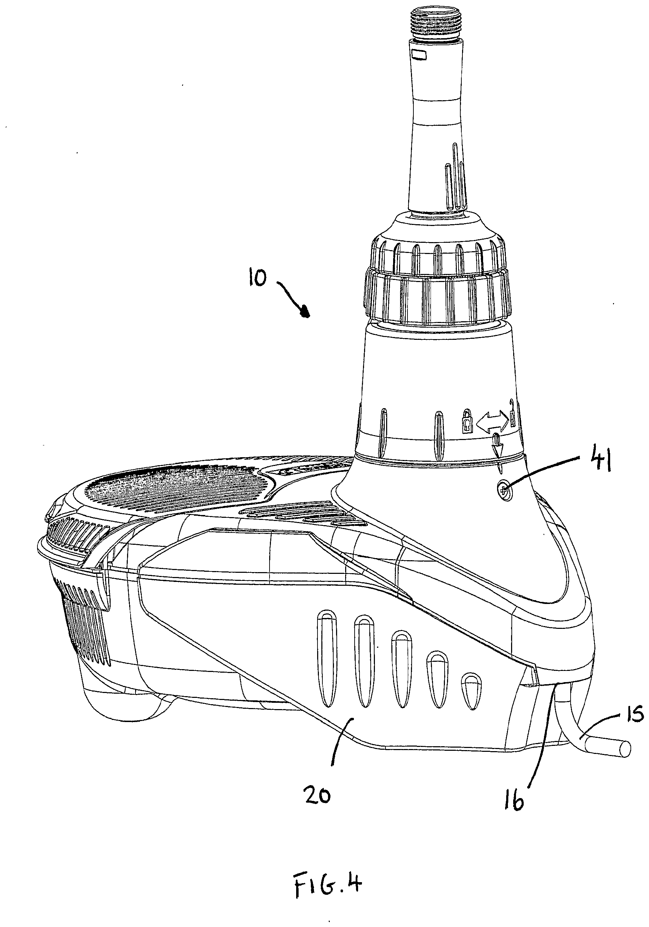

Taken together: the casing 72 of the pump 70; and the base 82, bayonet fitting 87 and quartz tube 86 of the UV lamp 80, form a sealed container in which all the electrical components of the pump unit 10 are housed in a watertight fashion. This allows the pump unit 10 to have a single electrical power lead, shown at 15 in Figure 4 running thereto. The power lead 15 enters the base portion 20 of the unit 10 via a suitable aperture 16, and from there enters the sealed container via an aperture (not shown) in the casing 72 of the pump 70.

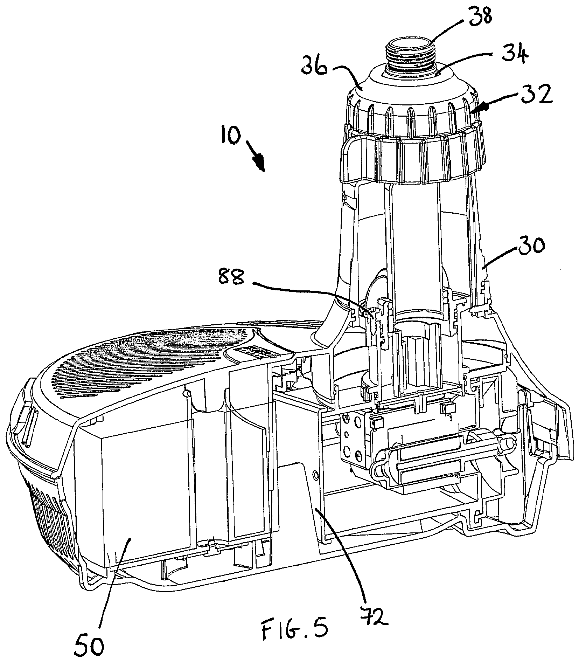

With reference to Figure 5, the upper end of the upper portion 30 of the pump unit 10 is formed into a socket 32 for receiving a ball joint 34. The ball joint 34 is a short length of plastic tube with the mid portion of its outer surface formed to resemble the outer surface of a sphere. A removable threaded collar 36 is provided for releasably and variably securing the ball joint 34 to the upper end of the upper portion 30, that upper end being formed with a cooperating thread. When secured in this way, an upper end 38 of the ball joint 34 projects beyond the collar 36. The upper end 38 of the ball joint 34 is threaded.

A fountain outlet 90 is provided for attachment to the threaded upper end 38 of the ball joint 34. This outlet 90 is shown attached in Figure 2. The fountain outlet 90 includes a pipe 92 with a nozzle portion 94 at one end. The other end of the pipe 92 is threaded and is for cooperating with the threaded upper end 38 of the ball joint 34 for releasable attachment to that end 38. The nozzle portion 94 is formed with holes therethrough arranged to direct water pumped therethrough into a jet of one or more streams.

The upper portion 30 of pump unit 10 includes a bypass outlet 100 towards its upper end, but below the fountain outlet 90, the ball joint 34 and the collar 36. The bypass outlet 100 includes an outlet aperture (not shown) in the upper casing 40b of the upper portion 30 that allows communication between the inside of the upper portion 30 and the outside. The bypass outlet 100 also includes a rotatable ring 102 that surrounds the upper portion 30 in the region of the outlet aperture. This region of the upper portion 30 is of reduced diameter, such that the ring 102 can be accommodated around that region so as to be flush with the surrounding outside surface of the upper portion 30. The ring 102 is formed and fitted to the upper portion 30 so as to be rotatable between a position in which the outlet aperture is almost closed by the ring 102 and a position in which the outlet aperture is open, and rotatable to positions between these two extremes.

For operation, the pump unit 10 is placed in a pond, such as a pond in the garden of a house. The pump unit 10 is positioned in the pond such that the outlet aperture in the upper portion 30 of the unit 10 is below the pond's water line and such that the nozzle 94 is above the water line. The electrical lead 15 is then connected, in this embodiment, to a mains source of electrical power. This causes the unit 10 to operate.

In operation, the electrical pump 70 and the UV lamp 80 receive power via the lead 15 and operate. The pump 70 reduces the pressure in its inlet below the ambient pressure of pond water surrounding the pump unit 10. This causes pond water to be drawn towards and into the inlet. Thus, water from the pond is drawn through the slots 46 in the base housing 40a, through the foam block 50 and the apertures 55 of the base portion 54 on which the block 50 sits, into the chamber 62 in the base portion 20 and, from there, into the pump 70 via its inlet. As the water is drawn along this flow path, the slots 46 in the base housing 40a serve to strain large pieces of solid matter from the water as it is drawn therethrough; and the foam serves to subsequently strain finer pieces of solid matter from the water. In the chamber 62, the water is drawn over and around the many surfaces of the biomedia 60 before entering the pump 70. Micro-organisms on the surfaces of the biomedia 60 tend to operate on the water to clean it further by feeding on ammonia and converting it into nitrite and then nitrate.

Water that passes into the operating electrical pump 70 exits the pump 70 via its outlet into the upper portion 30 of the pump unit 10. The pump 70 increases the pressure of water at its outlet to a pressure above the ambient pressure of water in the pond. Thus, water flows from the outlet, up the upper portion 30, around and past the operating UV lamp 80 to the upper end of the upper portion 30. From here, some of the water flows through the ball joint 34, up the pipe 92 of the fountain outlet 90 to exit the unit 10 in one or more jets via the nozzle 94, thereby forming a fountain of water before returning to the pond. The remainder of the water flows through the aperture of the bypass outlet 100 to thereby exit the unit 10 and rejoin the remainder of the water in the pond. As the water flows around and past the UV lamp 80, that lamp 80 exposes the water and its contents to UV light. The UV light tends to cause the cell walls of algae in the water to rupture, which in turn causes the algae to gather together in groups that either sink to the bottom of the pond or are caught by mechanical filtering.

The rotatable ring 102 of the bypass outlet 100 is rotatable to vary the amount of water that exits the pump unit 10 via that bypass outlet 100 so as to bypass the fountain outlet 90. Rotating the ring 102 to open the bypass outlet 100 to its fullest extent results in substantially all of the water from the pump 70 exiting the unit 10 via the bypass outlet 90. This feature may be used when only a small fountain of water is required. Alternatively, the ring 102 can be rotated to close the bypass outlet 100 to its fullest extent. This, however, does not completely close the bypass outlet 100, but still allows some water from the pump 70 to exit the unit 10 therethrough. This ensures that water can still flow through the unit 10 even if the nozzle 94 of the fountain outlet 90 were to become blocked. Selectively rotating the ring 102 to positions between these two extremes allows the height of the fountain of water produced by the unit 10 to be varied in a controlled manner.

In the event that the foam block 50 becomes clogged by solid matter trapped therein, an increased pressure differential is created across the valve 58 in tube 56 of the bypass member 52 on which the block 50 sits. This causes the valve 58 to open and to allow water to flow along the tube 56 and into the chamber 62 and the pump 70 in the base portion 20. Thus, the foam block 50 is bypassed when clogged. Again, this allows the pump unit 10 to continue to operate and, in particular, to continue to expose pond water to the UV lamp 80 when the foam block 50 is at least partially clogged.

Should the foam block 50 become blocked in the way described, it can be removed and either cleaned or replaced. To remove the foam block 50, the lid 42 of the base portion 20 is first removed from the remainder of that portion 20 by pressing on the two button portions 44 of the catches, and lifting the lid 42. This allows access to the foam block 50. The foam block 50, together with the bypass member 52 on which the foam block 50 sits, can then be removed from the base portion 20. The foam block 50 and the bypass member 52 would then be separated from one another in order to allow cleaning or replacement of the foam block 50. The pump unit 10 is reassembled by carrying out this process in reverse.

The UV bulb 84 may also be removed for, for example, replacement. It this is to be done, electrical power to the pump unit 10 should first be cut off. The unit 10 may then be removed from the pond. Access to the UV lamp 80 is gained by removing the upper housing 40b around the upper portion 30 of the unit 10. This part of the housing 40 is twist mountable onto the remainder of the housing 40 (i.e. the base housing 40a) and is easily removed once a locating screw (shown at 41 in Figure 4) is removed. The quartz tube 86 can then be removed by unscrewing the screw 88 in the bayonet fitting 87 and twisting that fitting 87. The UV bulb 84 can then be removed, replaced and the process reversed to re-assemble the pump unit 10.

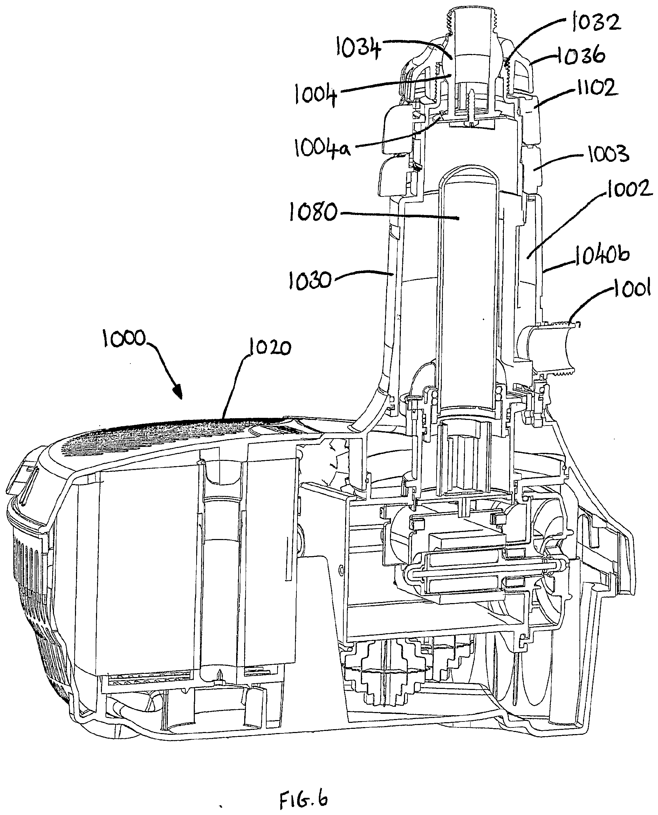

Figure 6 shows a pump unit 1000 which is similar to the pump unit 10 shown in Figures 1 to 5. The pump unit 1000 shown in Figure 6 is arranged for use in a larger pond than that shown in Figure 5 but in many respects is the same, or substantially the same, as the pond unit 10 shown in Figures 1 to 5. Therefore, detailed description of the majority of the pump unit 1000 shown in Figure 6 will be omitted and the same reference numerals are used to refer to the corresponding parts with the exception that 1000 is added to each reference numeral so that 20 becomes 1020 and so on.

The base portion 1020 of the pump unit 1000 shown in Figure 6 is basically the same as that shown in Figures 1 to 5 except that it has increased dimensions. Again this base portion 1020 is arranged to stand submerged in the water of a pond. Indeed, as is the case for the pump unit shown in Figures 1 to 5, the whole unit is arranged so as to be submersible in water with the tip of a fountain outlet (not shown in Figure 6) at or near the surface.

In the pump unit 1000 shown in Figure 6 there is again the cylindrical upper portion 1030 which is narrower than the squat cylindrical base portion 1020. In this pump unit 1000 a take off outlet 1001 is provided in the upper portion 1030. This take off outlet 1001 is arranged to allow a supply of filtered water to leave the pump unit 1000 via a suitable hose connected to the take off outlet 1001 and to be fed by the hose to a remote outlet, for example so as to form a waterfall feature.

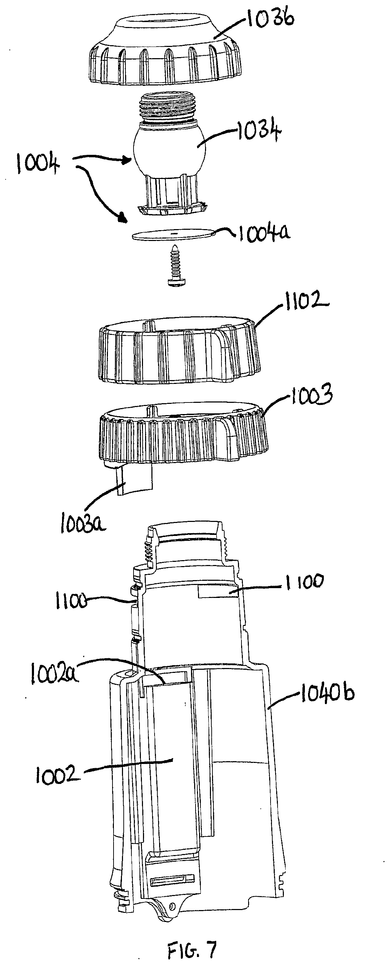

The pump unit 1000 also includes a flow control for controlling the flow which leaves the pump unit 1000 via the take off outlet 1001. As most clearly seen by reference to Figure 7 as well as Figure 6, a take off outlet conduit 1002 is provided on the inside surface of the upper housing 1040b of the upper portion 1030. This conduit 1002 has an inlet 1002a towards its upper end and feeds into the take off outlet 1001 at its lower end. By reference to Figure 6 it can be seen that the inlet 1002a to the conduit 1002 is at the downstream end of the UV lamp 1080 so that water entering the conduit 1002 to leave the pump unit 1000 via the take off outlet 1001 will have passed the UV lamp 1080.

In this pump unit 1000, as well as a rotatable ring or collar control 1102 for controlling a by-pass outlet 1100 for allowing water to by-pass the fountain outlet 1090 (in the same way as in the first pump unit shown in Figures 1 to 5), there is provided a second rotatable ring or collar control 1003 which is operable by a user to control the flow allowed out of the take off outlet 1001. The collar control 1003 for the take off outlet 1001 comprises a depending tongue 1003a which is best seen in Figure 7 and which is arranged to substantially blank off the inlet 1002a to the conduit 1002 if the collar 1003 has a rotational position which is such that the tongue 1003a is in register with the outlet 1002a. As can be seen by consideration of Figure 7, if the collar 1003 is moved away from register with the inlet 1002a, the inlet 1002a will be progressively opened until the tongue 1003 moves completely out of the way of the inlet 1002a.

Generally speaking in pump units of the type considered in this application it is not necessary to obtain a perfect seal between components such as the tongue 1003a and the opening 1002a to the conduit 1002. If some water is able to leak out through such a path this is not of great concern. What is desirable is the ability to control, at a general level, the water flow which is occurring.

It can be seen that the conduit 1002 is defined partially by the side wall of the housing 1040b and partially by a component which is mounted to that side wall. The conduit extends in a generally axial direction relative to the upper portion 1030 and also extends generally axially relative to the UV lamp 1080. The arrangement of the conduit 1002 is such that the water flow path passes the UV lamp in a first direction and then passes back along the conduit 1002 in an opposite direction before leaving via the take off outlet 1001. It will be noted that in this embodiment a confined conduit 1002 is provided which extends around only a relatively small part of the circumference of the housing 1040 of the upper portion 1030. In an alternative, an annular conduit could be provided by providing a cylindrical wall portion which fits inside the housing 1040b and which has one or more suitable inlets at its upper end to allow the ingress of water.

Fountain by-pass outlets 1100 of the second pump unit 1000 can also be seen in Figure 7, and as in the first pump unit 10, water flow through these is controlled by the respective collar control 1102. Rotation of this collar control 1102 serves to alter the proportion of the by-pass outlets 1100 which are blocked to water flow.

The pump unit 1000 shown in Figures 6 and 7 also comprises a modified form of ball joint assembly 1004 which includes a ball joint 1034 which is the same as that in the pump unit of Figures 1 to 5 and which is again received in a socket 1032 in the upper portion 1030 of the unit. In this unit however, a UV lamp obscuring disc 1004a is attached to the ball joint 1034 using a screw. This UV lamp obscuring disc 1004a serves to substantially prevent viewing of the UV lamp 1080 if the retaining collar 1036 is removed from the upper portion of the unit 1030. Furthermore, the inclusion of this obscuring disc 1004a also serves to capture the ball joint assembly 1004 in the upper portion of the unit. As can be best seen in Figure 6, the socket portion 1032 prevents movement of the ball joint assembly 1004 in one direction whereas the obscuring disc 1004a prevents movement of the assembly 1004 in the opposite direction. This means that not only does the obscuring disc 1004a blank out a possible line of sight to the UV lamp 1080 when the retaining collar 1036 is removed but also prevents removal of the ball joint assembly 1004 from the external side of the casing 1040b without first removing the obscuring disc 1004a.

Whilst in this embodiment the obscuring disc 1004a is fixed to the ball joint 1034 using a screw, in alternatives the obscuring disc may be push fitted or clipped onto the ball joint 1034.

## Claims

1. A pond pump unit (10) for placing in a pond, the unit comprising a housing (40) in which is housed an electrical pump (70) and an electrical UV lamp (80), wherein the unit is arranged such that the pump (70) can draw water from the pond and is operable to cause that water to flow past the UV lamp (80), so as to be exposed to UV light therefrom, before rejoining the pond, the unit being arranged so that the pump unit (10) is submersible in water and
**characterised in that** the unit further comprises a fountain outlet (90) comprising a pipe (92) with a nozzle portion (94) at one end for creating a fountain, and;
a bypass outlet (100) arranged to provide an outlet for at least some of the water drawn from the pond, so as to bypass the fountain outlet (90) and rejoin to the pond without forming a fountain wherein the unit is arranged so that the bypass outlet (100) receives water that has already flowed past the UV lamp (80), and;
a variable flow control means arranged to variably control the flow of water though the bypass outlet (100) and/or the fountain (90), and;
a biological filtering structure (60) arranged with a high surface area to volume ratio and arranged in the housing such that water drawn from the pond is caused to flow over and around the structure, and;
the unit further comprises a mechanical filtering means (50) and is arranged such that the pump (70) causes the water drawn from the pond to pass through those means such that the water is filtered thereby, and which further comprises a bypass for allowing water drawn from the pond to bypass (52) at least part of the mechanical filtering means (50) when water flow though that means is hindered by at least partial clogging thereof.

2. A pond pump unit according to claim 1 in which apertures (46) are formed in the housing of the unit through which water from the pond is drawn by the pump (70).

3. A pond pump unit according to claim 1 or claim 2 in which the electrical components of the pump (70) are contained in sealed container which is sealed against the ingress of water and the electrical components of the lamp (80) are contained in sealed container which is sealed against the ingress of water.

4. A pond pump unit according to claim 3 arranged such that electrical components of the pump (70) and of the UV lamp (80) are contained together in a sealed container in the housing, the sealing of the sealed container being against the ingress of water.

5. A pond pump unit according to claim 4 wherein the sealed container is arranged to have a single power lead extending therefrom to supply electrical power to both of the pump (70) and the lamp (80).

6. A pond pump unit according to claim 1 wherein the bypass (52) includes a valve (58),
which is arranged such that at least partial clogging of the filter means (50) increases a pressure difference across the valve such that the valve opens and allows water drawn from the pond to pass therethrough and to thereby bypass at least part of the filter means (50), and
wherein a bypass member is provided that defines the bypass flow path with the valve situated therein, and the valve comprises a thin flap of material that is resiliently biased in a closed position and is situated at an outlet side of the bypass flow path.

7. A pond pump unit according to claim 1 wherein the variable flow control means is user adjustable by means of a rotatable collar (102).

8. A pond pump unit according to any preceding claim wherein the housing further comprises an outlet structure that at least partly defines the fountain outlet (90), the outlet structure being moveable relative to the remainder of the housing so as to allow water exiting the unit from the fountain outlet to be directed in a preferred direction.

9. A pond pump unit according to any preceding claim in which the unit
comprises a takeoff outlet (1001) for supplying water via a hose to a remote outlet, and comprises
variable flow control means for variable control of water flow through the takeoff outlet.

10. A pond pump unit according to claim 9 in which the variable flow control means comprises a rotatable collar (1003).

11. A pond pump unit according to claim 9 in which the unit comprises a takeoff outlet conduit (1002) providing a passage from the downstream end of the UV lamp to the takeoff outlet, the takeoff outlet conduit (1002) having an inlet disposed at the downstream end of the UV lamp, and the variable flow control means comprising a closure member for closing, by controllable degrees, the inlet of the takeoff outlet conduit (1002).

## Patentansprüche

1. Teichpumpeneinheit (10) für ein Platzieren in einem Teich, wobei die Einheit ein Gehäuse (40) umfasst, in dem eine elektrische Pumpe (70) und eine elektrische UV-Lampe (80) untergebracht sind, wobei die Einheit derart angeordnet ist, dass die Pumpe (70) Wasser aus dem Teich entnehmen kann und betriebsfähig ist, um zu bewirken, dass Wasser an der UV-Lampe (80) vorbeiströmt, um UV-Licht davon ausgesetzt zu werden, bevor es mit dem Teich zusammentrifft, wobei die Einheit angeordnet ist, so dass die Pumpeneinheit (10) in Wasser eintauchbar ist und
**dadurch gekennzeichnet, dass** die Einheit ferner einen Springbrunnenauslass (90) umfasst, der ein Rohr (92) mit einem Düsenabschnitt (94) an einem Ende zum Erzeugen eines Springbrunnens umfasst, und;
einen Umgehungsauslass (100), der angeordnet ist, um einen Auslass für wenigstens ein wenig Wasser bereitzustellen, das aus dem Teich entnommen wird, um den Springbrunnenauslass (90) zu umgehen und mit dem Teich zusammenzutreffen, ohne einen Springbrunnen auszubilden, wobei die Einheit angeordnet ist, so dass der Umgehungsauslass (100) Wasser aufnimmt, das bereits an der UV-Lampe (80) vorbeigeströmt ist, und;
ein variables Strömungssteuermittel, das angeordnet ist, um die Wasserströmung durch den Umgehungsauslass (100) und/oder den Springbrunnen (90) variabel zu steuern, und;
eine biologische Filterstruktur (60), die mit einem hohen Oberflächenbereich zu Volumenverhältnis angeordnet ist und in dem Gehäuse derart angeordnet ist, dass bewirkt wird, dass Wasser, das aus dem Teich entnommen wird, über und um die Struktur strömt, und;
die Einheit ferner ein mechanisches Filtermittel (50) umfasst und derart angeordnet ist, dass die Pumpe (70) bewirkt, dass das Wasser, das aus dem Teich entnommen wird, durch dieses Mittel derart führt, dass das Wasser dadurch gefiltert wird, und das ferner eine Umgehung zum Ermöglichen von Wasser, das aus dem Teich entnommen wird, wenigstens einen Teil des mechanischen Filtermittels (50) zu umgehen (52), wenn die Wasserströmung durch dieses Mittel durch wenigstens teilweises Verstopfen davon behindert wird, umfasst.

2. Teichpumpeneinheit nach Anspruch 1, in der in dem Gehäuse der Einheit Öffnungen (46) ausgebildet sind, durch die Wasser aus dem Teich durch die Pumpe (70) entnommen wird.

3. Teichpumpeneinheit nach Anspruch 1 oder 2, in der die elektrischen Komponenten der Pumpe (70) in einem abgedichteten Behälter enthalten sind, der gegen das Eindringen von Wasser abgedichtet ist, und die elektrischen Komponenten der Lampe (80) in einem abgedichteten Behälter enthalten sind, der gegen das Eindringen von Wasser abgedichtet ist.

4. Teichpumpeneinheit nach Anspruch 3, die derart angeordnet ist, dass elektrische Komponenten der Pumpe (70) und der UV-Lampe (80) zusammen in einem abgedichteten Behälter in dem Gehäuse enthalten sind, wobei die Abdichtung des abgedichteten Behälters gegen das Eindringen von Wasser erfolgt.

5. Teichpumpeneinheit nach Anspruch 4, wobei der abgedichtete Behälter angeordnet ist, um eine einzelne Leistungszuführung aufzuweisen, die sich davon erstreckt, um sowohl die Pumpe (70) als auch die Lampe (80) mit elektrischer Leistung zu versorgen.

6. Teichpumpeneinheit nach Anspruch 1, wobei die Umgehung (52) ein Ventil (58) einschließt,
das derart angeordnet ist, dass wenigstens teilweises Verstopfen des Filtermittels (50) eine Druckdifferenz über dem Ventil derart erhöht, dass sich das Ventil öffnet und Wasser ermöglicht, das aus dem Teich entnommen wird, dahindurch zu führen, um dadurch wenigstens einen Teil des Filtermittels (50) zu umgehen, und
wobei ein Umgehungselement bereitgestellt ist, das den Umgehungsströmungsweg mit dem darin gelegenen Ventil definiert, und das Ventil eine dünne Materialklappe umfasst, die in eine geschlossene Position elastisch vorgespannt ist und an einer Auslassseite des Umgehungsströmungswegs gelegen ist.

7. Teichpumpeneinheit nach Anspruch 1, wobei das variable Strömungssteuermittel mittels eines drehbaren Kragens (102) von einem Benutzer einstellbar ist.

8. Teichpumpeneinheit nach einem der vorhergehenden Ansprüche, wobei das Gehäuse ferner eine Auslassstruktur umfasst, die wenigstens teilweise den Springbrunnenauslass (90) definiert, wobei die Auslassstruktur relativ zu dem Rest des Gehäuses bewegbar ist, um Wasser zu ermöglichen, das aus der Einheit aus dem Springbrunnenauslass austritt, in eine bevorzugte Richtung gelenkt zu werden.

9. Teichpumpeneinheit nach einem der vorhergehenden Ansprüche, in der die Einheit einen Abnahmeauslass (1001) für ein Versorgen von Wasser über einen Schlauch zu einem entfernten Auslass umfasst, und Folgendes umfasst:
variables Strömungssteuermittel für eine variable Steuerung der Wasserströmung durch den Abnahmeauslass.

10. Teichpumpeneinheit nach Anspruch 9, in der das variable Strömungssteuermittel einen drehbaren Kragen (1003) umfasst.

11. Teichpumpeneinheit nach Anspruch 9, in dem die Einheit eine Abnahmeauslassleitung (1002) umfasst, die einen Durchgang von dem stromabwärtigen Ende der UV-Lampe zu dem Abnahmeauslass bereitstellt, wobei die Abnahmeauslassleitung (1002) einen Einlass aufweist, der an dem stromabwärtigen Ende der UV-Lampe eingerichtet ist, und wobei das variable Strömungssteuermittel ein Schließelement für ein Schließen des Einlasses der Abnahmeauslassleitung (1002) durch steuerbare Grade umfasst.

## Revendications

1. Unité de pompage de bassin (10) destinée à être placée dans un bassin, l'unité comprenant un boîtier (40) dans lequel sont logées une pompe électrique (70) et une lampe UV électrique (80), dans laquelle l'unité est agencée de sorte que la pompe (70) puisse tirer de l'eau depuis le bassin et permettre d'amener l'eau à s'écouler en passant par la lampe UV (80), de manière à être exposée à une lumière ultraviolette provenant de cette-dernière, avant de rejoindre le bassin, l'unité étant agencée de sorte que l'unité de pompage de bassin (10) est submersible dans l'eau et
**caractérisée en ce que** l'unité comprend en outre une sortie de fontaine (90) comprenant un tuyau (92) avec une partie de buse (94) à une extrémité pour créer une fontaine, et
une sortie de dérivation (100) agencée pour fournir une sortie pour au moins une partie de l'eau tirée à partir du bassin, de manière à contourner la sortie de fontaine (90) et rejoindre le bassin sans former de fontaine, dans laquelle l'unité est agencée de manière à ce que la sortie de dérivation (100) reçoive de l'eau qui s'est déjà écoulée au-delà de la lampe UV (80), et ;
un moyen de régulation d'écoulement variable agencé pour réguler de manière variable l'écoulement d'eau à travers la sortie de dérivation (100) et/ou la fontaine (90), et
une structure de filtration biologique (60) agencée avec un rapport surface/volume élevé et agencée dans le boîtier de sorte que l'eau tirée à partir du bassin soit amenée à s'écouler sur et autour de la structure, et ;
l'unité comprend en outre un moyen de filtration mécanique (50) et est agencée de sorte que la pompe (70) amène l'eau tirée à partir du bassin à passer à travers ce moyen de sorte que l'eau soit filtrée par ce biais, et qui comprend en outre un conduit de dérivation pour permettre à l'eau provenant du bassin de contourner (52) au moins une partie du moyen de filtration mécanique (50) lorsque de l'eau s'écoulant à travers ce moyen est entravée par un engorgement au moins partiel de ce dernier.

2. Unité de pompage de bassin selon la revendication 1, dans laquelle des ouvertures (46) sont formées dans le boîtier de l'unité à travers lequel de l'eau provenant du bassin est tirée par la pompe (70).

3. Unité de pompage de bassin selon la revendication 1 ou la revendication 2, dans laquelle les composants électriques de pompe (70) sont contenus dans un contenant étanché qui est rendu étanche à l'afflux d'eau et les composants électriques de la lampe (80) sont contenus dans un contenant étanché qui est rendu étanche à l'afflux d'eau.

4. Unité de pompage de bassin selon la revendication 3, agencée de sorte que des composants électriques de la pompe (70) et de la lampe UV (80) sont contenus ensemble dans un contenant étanche dans le boîtier, le contenant étanche étant étanche à l'afflux d'eau.

5. Unité de pompage de bassin selon la revendication 4, dans laquelle le conteneur étanché est agencé pour avoir un seul câble d'alimentation s'étendant à partir de celui-ci pour fournir de l'énergie électrique à la fois de la pompe (70) et à la lampe (80).

6. Unité de pompage de bassin selon la revendication 1, dans laquelle le conduit de dérivation (52) comprend une soupape (58), qui est agencée de telle sorte qu'un engorgement au moins partiel du moyen de filtration (50) augmente une différence de pression sur la soupape de sorte que la soupape s'ouvre et permette le passage de l'eau tirée depuis le bassin à travers cette dernière et de contourner ainsi au moins une partie du moyen de filtration (50), et
dans lequel un élément de dérivation est fourni, qui définit le trajet d'écoulement de dérivation avec la soupape située en son sein, et la soupape comprend un mince rabat de matériau qui est sollicité de manière élastique dans une position fermée et est situé au niveau d'un côté de sortie du trajet d'écoulement de dérivation.

7. Unité de pompage de bassin selon la revendication 1, dans laquelle le moyen de régulation d'écoulement variable peut être réglé par l'utilisateur au moyen d'une bague rotative (102).

8. Unité de pompage de bassin selon l'une quelconque des revendications précédentes, dans laquelle le boîtier comprend en outre une structure de sortie qui définit au moins partiellement la sortie de fontaine (90), la structure de sortie étant mobile par rapport au reste du boîtier de manière à permettre que l'eau sortant de l'unité par la sortie de fontaine soit dirigée dans une direction préférée.

9. Unité de pompage de bassin selon l'une quelconque des revendications précédentes, dans laquelle l'unité comprend une sortie de prélèvement (1001) pour fournir de l'eau via un tuyau à une sortie distante, et comprend
un moyen de régulation d'écoulement variable pour une régulation variable de l'écoulement d'eau à travers la sortie de prélèvement.

10. Unité de pompage de bassin de bassin selon la revendication 9, dans laquelle le moyen de régulation d'écoulement variable comprend une bague rotative (1003).

11. Unité de pompage de bassin selon la revendication 9, dans laquelle l'unité comprend une conduite de sortie de prélèvement (1002) fournissant un passage à partir de l'extrémité aval de la lampe UV pour la sortie de prélèvement, la conduite de sortie de prélèvement (1002) comprenant une entrée disposée au niveau de l'extrémité aval des lampes UV, et le moyen de régulation d'écoulement variable comprenant un élément de fermeture pour fermer, selon des degrés réglables, l'afflux de la conduite de sortie de prélèvement (1002).
